# EUROPEAN PATENT APPLICATION

(11) **EP 1 310 210 A2**
(43) Date of publication of application: **14.05.2003**
(21) Application number: 02102533.3
(22) Date of filing: 05.11.2002
(51) Int. Cl.: A61B 5/022

(54) **Method and assembly for identifying a measuring cuff**

(30) Priority: 09.11.2001 FI 20012174
(71) Applicant: Instrumentarium Corporation, 00510 Helsinki (FI)
(72) Inventor: Martikainen, Antti, 01830 Lepsämä (FI)
(74) Representative: Valkeiskangas, Tapio

(57) **Abstract**

The invention relates to a method and an assembly for identifying a measuring cuff from among different available measuring cuffs in non-invasive blood pressure measurement, in which method measuring electronics and safety electronics as well as a first identification arrangement and a second identification arrangement are used, and in which method the first and the second identification arrangement are used independently from each other. In order to achieve reliable identification, second means for the second identification arrangement are arranged to operate based on the cooperation of a magnet (10) and a circuit (11) identifying a magnetic field.

## Description

The invention relates to a method of identifying a measuring cuff from among different available measuring cuffs in non-invasive blood pressure measurement, in which method measuring electronics and safety electronics as well as a first identification arrangement and a second identification arrangement are used, and in which method the first and the second identification arrangement are used independently from each other. The invention also relates to an assembly for identifying a measuring cuff.

Device arrangements in which a blood pressure measuring cuff is selected with a selecting switch arranged in the device, for instance according to whether the object of measurement is an adult or a new-born baby, have been known and used in the field for a long time. In other words, the selecting switch is used manually in the selection of the operation mode of the equipment according to whether the measurement is performed for an adult or for a child. In principle, the above-mentioned equipment functions well, but there is always a risk that the user forgets to change the position of the selecting switch, for instance when changing a measuring cuff intended for adults to a measuring cuff intended for children. Such an error may, in the worst case, cause severe injuries for a new-born baby.

For the above-mentioned reasons, different identification arrangements have been provided in the field, with which a measuring cuff attached to measuring equipment can be identified automatically. Examples of the solutions known in the field include, for instance, the solutions of US patents 5 003 981 and 5 060 654.

Further, the solution of US patent 5 572 992 can be mentioned as an example of general technology relating to identification.

In principle, the above-mentioned prior art solutions function well and have essentially eliminated drawbacks of previous technology. However, recent standards concerning non-invasive blood pressure measurement require not only the use of measuring electronics but also the use of safety electronics controlling the operation, so that previous solutions do not, in this respect, conform to the present standards.

An object of the invention is to provide a method and an assembly for identifying a measuring cuff correctly also in the case of one error in non-invasive blood pressure measurement. This has been achieved by means of the invention. The method according to the invention is characterized by the second identification arrangement being based on cooperation of a magnet and a circuit identifying a magnetic field. An assembly according to the invention, in turn, is characterized in that second means for the second identification arrangement are arranged to operate based on the cooperation of a magnet and a circuit identifying a magnetic field.

An advantage of the invention is, above all, that the drawbacks of different mechanical and optical switches can be efficiently eliminated, in other words the circuit identifying the magnetic field can be positioned in a connector in such a way that gas tightness remains as it is without a sensor. Getting dirty or wearing in an ordinary way does not affect the operation of the circuit identifying the magnetic field either, so that the reliability of the assembly according to the invention is good. Further, an advantage of the invention is its simplicity, so that the introduction and use of the invention are inexpensive.

The invention will now be described in greater detail with reference to the embodiments shown in the attached drawings, of which

Figure 1 illustrates a principled view of a non-invasive blood pressure measurement situation;

Figure 2 illustrates a principled view of an essential detail of an embodiment of the assembly according to the invention;

Figures 3, 4 and 5 illustrate principled views of different embodiments of the invention; and

Figure 6 illustrates a block diagram of an embodiment of the assembly according to the invention.

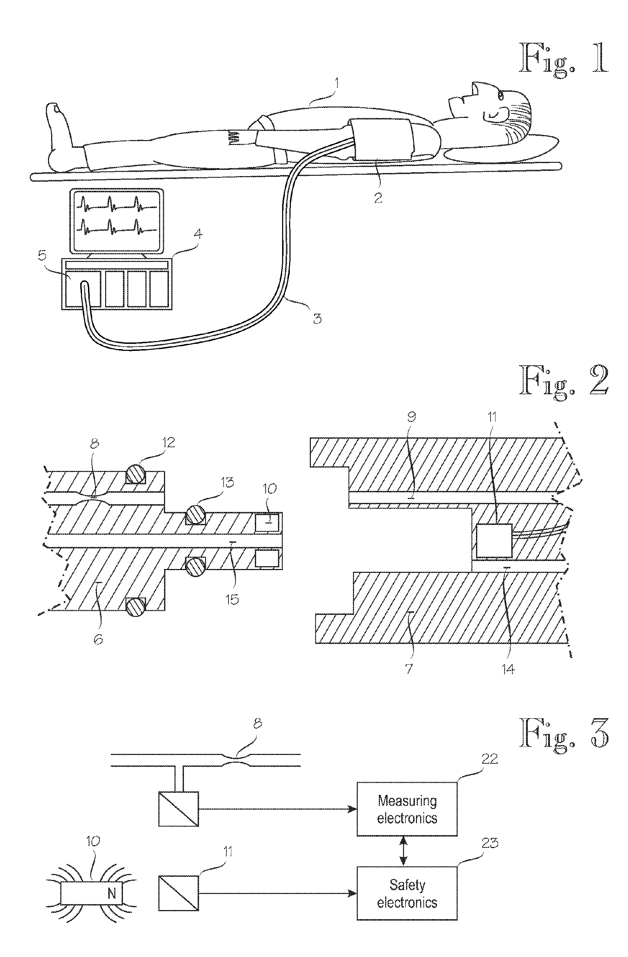

Figure 1 illustrates a principled situation where blood pressure is measured in a non-invasive manner. Figure 1 shows a patient indicated with reference numeral 1. Reference numeral 2 denotes a blood pressure measuring cuff. Reference numeral 3 indicates a measuring hose from the module of the blood pressure measuring device to the cuff 2. Reference numeral 4 indicates the patient's monitoring device, the reference numeral 5 denoting a blood pressure measuring module in the monitoring device.

The measuring cuff 2 is attached to the patient's one arm, or alternatively, one thigh. The measurement is performed at regular intervals, for example at intervals of five minutes. The monitoring device 4 monitors the measurement in accordance with the measuring interval defined for it. A safety electronics microprocessor controls all the time that the pressure limits or allowed measuring times/measuring intervals defined in the standards are not exceeded.

The invention relates to the measurement shown in Figure 1. Figure 2 shows the essential details of the assembly according to the invention in larger scale. Reference numeral 6 denotes a measuring cuff connector, i.e. a connector in the measuring cuff, whereas reference 7 shows a measuring device/module connector, which is in the module 5, for example. Figure 2 thus illustrates a principled connecting point by means of which the measuring cuff 2 is connected to the module 5.

In accordance with the invention, identification of the measuring cuff utilizes two methods independently from each other. The system comprises two electronics parts, i.e. measuring electronics and safety electronics.

The measuring electronics utilize flow restriction limiting the flow rate for the identification of the measuring cuff. Such a restriction point is indicated by reference numeral 8 in Figure 2. The restriction point is in connection with a side flow channel of the connector of the measuring cuff 2. The side flow channel in the connector 7 is shown by reference numeral 9. The restriction point is in this example arranged in connection with a neonatal cuff. In this example, there is no restriction point in a measuring cuff intended for adults.

Reference numerals 12 and 13 indicate O-rings. The O-ring 12 seals the connection towards the atmospheric air, and the O-ring 13 keeps the main flow and the side flow separated from each other. Reference numeral 14 indicates the main flow channel in the connector of the measuring device, whereas reference numeral 15 denotes the main flow in the connector of the measuring cuff.

In accordance with the invention, the safety electronics utilize in the identification a magnet 10 in the measuring cuff connector and a circuit 11 identifying the magnetic field in the measuring device/module connector. The circuit 11 identifying the magnetic field may be a Hall sensor, for example, or any other sensor based on inductive identification of the magnetic field.

The arrangement according to Figure 2 can be implemented in a principled way in accordance with Figures 3, 4 and 5. In Figures 3, 4 and 5, the corresponding points have the same reference numerals as in Figures 1 and 2. Reference numeral 22 generally indicates measuring electronics, and reference numeral 23 correspondingly indicates safety electronics.

The system functions in principle in the same way. If the circuit 11 identifying a magnetic field detects a magnet 10 in the connector of the measuring cuff 2, the cuff in question is a neonatal cuff. In such a case, the safety electronics control the pressure and time limits in accordance with the limits defined for neonatal patients. If no magnet 10 is detected, the limits defined for adults are used. If, in turn, the measuring electronics detects flow restriction in one measuring cuff hose or in the side flow channel of the measuring cuff connector, the cuff in question is a neonatal cuff, and the measurement is performed in accordance with the limits defined for neonatal patients in the standards. If no flow restriction is detected, the limits defined for adults are used. The measuring electronics and safety electronics compare the above-mentioned cuff detection information, and if a difference in the information is detected, no new measurement is started but instead, an error code is given to locate the error, or if required, the measurement is performed in accordance with the safer neonatal limits. The information can be transferred between the different parts of the system in a plurality of ways, as shown in Figures 3 to 5, but the basic idea is as described above.

The above-mentioned identification of the measuring cuff based on identification of a magnetic field and on a magnet is very reliable. Getting dirty or wearing in an ordinary way does not deteriorate the reliability. If a Hall sensor is used as the circuit identifying the magnetic field, a further advantage is that the solution based on the Hall sensor and the magnet makes copying of a product part more difficult. Copying is relatively difficult, because the magnetization direction of the magnet to be mounted on the connector of the measuring cuff must be correct in order to identify it. Copying is made more difficult also by the flow restriction in the connector of the measuring cuff. These solutions together and in the same connector make copying even more difficult. The above aspects add to the patients' safety, because no pirate parts of poor quality can be used. Double identification also prevents certain malfunctions. For example, if the O-ring attending to the sealing of the measuring cuff connector is broken, the magnetic identification prevents the pumping of adult pressures to a neonatal cuff.

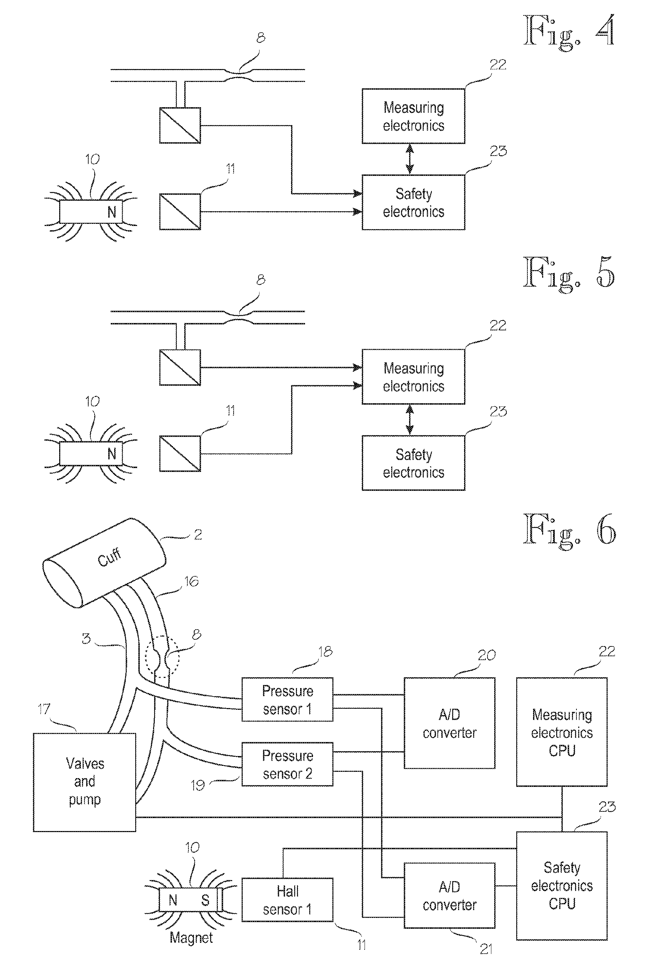

The invention will now be further described with reference to a more detailed example shown in Figure 6. Figure 6 shows a block diagram of non-invasive blood pressure measurement. The same reference numerals as in Figures 1 and 2 are used in the corresponding points in Figure 6. Reference numeral 16 indicates in Figure 6 the pumping hose of a measuring cuff. Reference numeral 17 indicates valves and a pump, while reference numeral 18 indicates the pressure sensor of the measuring hose and reference numeral 19 indicates the pressure sensor of the pumping hose. Reference numeral 20 shows the A/D converter of the measuring electronics, reference numeral 21 showing the A/D converter of the safety electronics. Reference numeral 22 indicates in Figure 6 the CPU of the measuring electronics, and reference numeral 23 indicates the CPU of the safety electronics.

The valves and the pump 17 may be connected to the same pressure delivery device as the pressure sensors 18, 19, or they can be separate and connected to each other with hoses. The signal from the pressure sensor is taken to the A/D converter either directly or amplified, depending on the sensor type. The pressure signal that has been converted into digital is taken from both pressure sensors 18, 19 to the microprocessor 22 of the measuring electronics and to the microprocessor 23 of the safety electronics. The A/D converter may be a separate part or integrated inside the microprocessor, depending on the device used. In this example, a Hall sensor has been used as the circuit 11 identifying the magnetic field. The signal from the Hall sensor 11 is directly digital, so that no A/D converter is needed with this signal between the sensor and the microprocessor. The flow restriction 8 and the magnet 10 are in this example positioned in the connector 6 of the measuring cuff 2. The flow restriction may also be positioned in the hose of the measuring cuff. The flow restriction and the magnet exist in this example only in the hose/connector of a neonatal cuff. There is neither flow restriction nor magnet in the hoses and connectors of other cuffs, such as cuffs intended for adults. The processors of the measuring electronics and the safety electronics compare their identification information, and if differences are detected, the system gives an error signal, or operation is continued at a safe level, as noted above.

The above-described application example is not intended to limit the invention in any way, but the invention can be modified completely freely within the scope of the claims. Thus, it is obvious that the assembly of the invention and its details need not be precisely as described in the figures, but other solutions are also feasible. In the example of the figures, the magnet is arranged in connection with a neonatal cuff. This is not, however, the only possibility, because in accordance with the basic idea of the invention, the magnet may be positioned in a measuring cuff intended for adults, for instance, in which case there is no identification magnet in the neonatal cuff. It is also to be noted that although the invention has been described in the above examples with reference to the identification of a neonatal cuff, it is obvious that the invention is not limited to this aspect in any way. The invention can naturally be used in the identification of any measuring cuff. Instead of identification based on flow restriction, another way of identification can naturally be used; what is essential is that the assembly utilizes two parallel ways of identification, one of which is based on the cooperation of a magnet and a circuit identifying a magnetic field.

## Claims

1. A method of identifying a measuring cuff from among different available measuring cuffs in non-invasive blood pressure measurement, in which method measuring electronics and safety electronics as well as a first identification arrangement and a second identification arrangement are used, and in which method the first and the second identification arrangement are used independently from each other, **characterized by** the second identification arrangement being based on cooperation of a magnet (10) and a circuit (11) identifying a magnetic field.

2. A method according to claim 1, **characterized by** the second identification arrangement being used in connection with safety electronics.

3. A method according to claim 1, **characterized by** the second identification arrangement being used in connection with measuring electronics.

4. A method according to any one of claims 1 to 3, **characterized by** the magnet (10) being arranged in a measuring cuff connector (6), and the circuit (11) identifying the magnetic field being arranged in a measuring device/module connector (7).

5. A method according to claim 4, **characterized by** the magnet (10) being arranged in the connector of a neonatal cuff.

6. A method according to any one of claims 1 to 5, **characterized by** the first identification arrangement being based on flow restriction (8) limiting the flow rate.

7. An assembly for identifying a measuring cuff from among different available measuring cuffs in non-invasive blood pressure measurement, which assembly comprises measuring electronics and safety electronics as well as first means for a first identification arrangement and second means for a second identification arrangement, whereby the first and the second identification arrangement are arranged to be used independently from each other, **characterized in that** the second means for the second identification arrangement are arranged to operate based on the cooperation of a magnet (10) and a circuit (11) identifying a magnetic field.

8. An assembly according to claim 7, **characterized in that** the second identification arrangement is arranged to be used in connection with safety electronics.

9. An assembly according to claim 7, **characterized in that** the second identification arrangement is arranged to be used in connection with measuring electronics.

10. An assembly according to any one of claims 7 to 9, **characterized in that** the magnet (10) is arranged in a measuring cuff connector (6) and the circuit (11) identifying the magnetic field is arranged in a measuring device/module connector (7).

11. An assembly according to claim 10, **characterized in that** the magnet (10) is arranged in the connector of a neonatal cuff.

12. An assembly according to any one of claims 7 to 11, **characterized in that** the first identification arrangement is arranged to be based on flow restriction (8) limiting the flow rate.

13. An assembly according to any one of claims 7 to 12, **characterized in that** the circuit (11) identifying the magnetic field is a device identifying the magnet inductively.

14. An assembly according to any one of claims 7 to 12, **characterized in that** the circuit (11) identifying the magnetic field is a Hall sensor.
